# EUROPEAN PATENT APPLICATION

(11) **EP 4 706 611 A2**
(43) Date of publication of application: **11.03.2026**
(21) Application number: 25221569.4
(22) Date of filing: 15.10.2021
(51) Int. Cl.: A61F 13/0203

(54) **A DERMATOLOGIC TREATMENT APPARATUS**

(30) Priority: 15.10.2020 GB 202016376; 06.07.2021 GB 202109763
(62) Divisional of application: 21807203.1
(71) Applicant: IP - Smart Ltd, Leamington Spa, Warwickshire CV32 4EA (GB)
(72) Inventor: Herbert, Kevin, Gloucester, GL3 2AP (GB)
(74) Representative: Wynne-Jones IP Limited

(57) **Abstract**

A dermatologic treatment apparatus (1) comprising a light emitting part (2), for emitting light for dermatological treatment. The apparatus (1) further comprises a skin treatment dressing (3). The skin treatment dressing (3) is a bioactive dressing and is arranged to adhere to a user's skin, during use. The skin treatment dressing (30 is arranged with the light emitting part (2) such that, in use, light from the light emitting part (2) passes to the skin treatment dressing (3). The skin treatment dressing (3) is configured such that the light from the light emitting part (2) passes through the skin treatment dressing (3) to a user's skin (2).

## Description

### Field of the Invention

The present invention relates to a dermatologic treatment apparatus, a dermatologic treatment apparatus assembly, a method of dermatological treatment, a method of manufacture of a dermatologic treatment apparatus and a kit of parts. In particular, but not exclusively, the invention relates to a light-based, self-contained adhesive dermatologic treatment apparatus.

### Background of the Invention

The treatment of dermatological conditions, such as acne and rosacea, can be considered to take one of two approaches - either a Skincare approach using over-the-counter, active ingredients in the form of creams or lotions or impregnated hydrocolloid dressings or through Skin-tech approaches for example phototherapy, electromechanical abrasive devices or ultrasound or electrical stimulation devices.

Skincare typically is quickly applied and requires no specialist knowledge but requires a consistent and continuous approach to treatment. Active ingredients typically require at least 2 or 3 skin growth cycles (56 to 84 days) to show any noticeable effects, and often a user will cease treatment with a particular skincare product long before it has had sufficient time to provide a noticeable effect on the underlying skin. When used correctly however, skincare can provide noticeable improvements for dermatological conditions.

A problem in the treatment of dermatological conditions, such as acne and rosacea with skincare is that creams and lotions can alter the characteristics of the skins defensive barrier (the skin mantle) and/or the skin biome and this can result in inflammation and slow down the healing process of a dermatological condition. This is particularly true where a user applies multiple skin care products during their routine.

Recent advances in skincare have seen the use of hydrocolloid dressings for the treatment of dermatological problems. These contain medication to further aid the treatment process relying on the absorption properties of the dressing or the use of micro-dart protrusions to increase active absorption efficiency. These provide the advantage of occluding the region undergoing treatment and prevent the user picking or otherwise interfering with a skin condition which might otherwise slow down the healing process.

Skintech typically uses electronic or electromechanical devices which require use for a longer period then skincare and occasionally some specialist knowledge is required to ensure an area is neither under nor over treated. For example, phototherapy requires that sufficient light dosage be applied to an area; undertreatment may result in insufficient destruction of bacteria, overtreatment may result in increased skin inflammation. Initial results are typically seen quickly, for example blue light phototherapy can reduced the quantity of bacteria on the skin dramatically within a week of use, however long-term results can only be obtained with continuous use. For example, in blue light phototherapy the reduction of bacteria is only temporary, and bacteria levels will return to normal once treatment is ceased.

The inventors have identified a need for the long term benefits and ease of use of skincare with the immediate results of skintech.

The present invention seeks to address or mitigate at least some of the above mentioned problems. Alternatively, or additionally, it is an object of this invention to provide an apparatus and method for the treatment of a dermatological condition which provides an immediate result and a sustained result.

Alternatively, or additionally, the present invention seeks to provide an improved dermatologic treatment apparatus. Alternatively, or additionally, the present invention seeks to provide an improved dermatologic treatment apparatus assembly. Alternatively, or additionally, the present invention seeks to provide an improved method of dermatological treatment. Alternatively, or additionally, the present invention seeks to provide an improved method of manufacture of a dermatologic treatment apparatus.

### Summary of the Invention

According to a first aspect of the invention there is provided a dermatologic treatment apparatus comprising:
a light emitting part, for emitting light for dermatological treatment;
wherein the apparatus further comprises a skin treatment dressing,
the skin treatment dressing is a bioactive dressing;
the skin treatment dressing is arranged to adhere to a user's skin, during use;
the skin treatment dressing is arranged with the light emitting part such that, in use, light from the light emitting part passes to the skin treatment dressing;
and wherein the skin treatment dressing is configured such that the light from the light emitting part passes through the skin treatment dressing to a user's skin.

The bioactive skin treatment dressing may occlude the region undergoing treatment, thereby preventing the user picking or otherwise interfering with a skin condition which might otherwise slow down the healing process, whilst allowing light, from the light emitting part, to pass through it to provide dermatological treatment. Accordingly the apparatus may provide the advantages of both a skin treatment dressing and of phototherapy. In this respect, the apparatus may provide the long term benefits and ease of use of a skin treatment dressing with the immediate results of phototherapy.

In this respect, applying both light for dermatological treatment and a bioactive dressing, to the skin, provides a synergistic technical effect, for a number of different phototherapeutic treatments, that use different colours/wavelengths of light.

In one example, Propionibacterium acnes (p.acnes) is a Gram-positive, microaerophilic bacterium and plays a major role in acne vulgaris by causing an inflammatory response in the skin. P.acnes release porphyrins, a group of organic compounds naturally occurring substances in the body, arising during the synthesis of haem in red blood cells. Porphyrins are pigmented compounds and when they absorb blue light (wavelength of 407-420 nm) they produce a radical. A prominent feature of radicals is that they have extremely high chemical reactivity and can inflict damage on nearby cells. When a radical is produced within (or around) p.acne bacteria the reactivity destroys the p.acnes bacteria. Acne vulgaris however forms from a complex chain of biological events, and simply removing p.acnes bacteria is insufficient to prevent acne reformation. While blue light can reduce the levels of bacteria within an inflamed pore, the environment that causes that bacteria is still the same and p.acnes will rapidly develop again unless that environment is changed.

The bioactive dressing (e.g. a hydrocolloid dressing) changes the pore environment and provides topical keratolytics to break down and help remove skin cells and skin oil from a blocked pore - this then allows air back into the pore and creates a hostile oxygen rich environment for p.acnes. By combining blue light with topical keratolytics, from a bioactive dressing, it is possible to obtain a synergistic effect with respect to reducing the symptoms of acne vulgaris, as this simultaneously allows for a lower keratolytic concentration with consecutive treatments (in short, by removing bacteria reduces inflammation which allows the keratolytic to function more effectively within a pore and vice versa by reducing the density of a pore bung, along with the antibacterial effect of some keratolytics like salicylic acid, allows a reduction in the amount of bacteria present in the pore which then results in a greater reduction overall by blue light).

As a further example, red light (wavelength of 585 to 830, perferably 633 nm) stimulates the production of collagen, elastin, and fibroblasts. Irradiation with a 633-nm light increases the synthesis of type 1 procollagen, decreases the expression of matrix metalloproteinase (MMP)1 and MMP2 in skin fibroblasts and decreases the expression levels of inflammatory genes, such has cyclooxygenase-2 (COX-2), and interleukin-1-α (IL-1α) in keratinocytes). The production of collagen is very complex but requires a complex fuel of vitamin C, peptides and simple sugars. Vitamin C above all is the essential cofactor for the two enzymes required for collagen synthesis: prolyl hydroxylase (to stabilize the collagen molecule) and lysyl hydroxylase (to give structural strength cross-linking). It has been found that bioavailability of Vit C in skin is inadequate when administered orally. Vitamin C applied topically (for example in a cream) has low bioavailability due to the fast oxidation process.

However, a bioactive dressing (e.g. a hydrocolloid dressing) allows gradual release of an active agent (e.g. vitamin C) to overcome the problem of its low bioavailability. For example, by providing vitamin C in a hydrocolloid, and applying red light, a synergistic effect occurs wherein the the synthesis of type 1 procollagen is increased greater than either the application of a topical vitamin C or red light alone.

Bioactive dressings are dressings that deliver substances active in wound healing, by delivery of one or more bioactive compounds, or constructed from materials having endogenous activity. These materials include hydrocolloids, alginates, collagens, chitosan, chitin, derivatives from chitosan or chitin and biotextiles.) In embodiments of the invention, the bioactive dressing comprises one or more active agents.

Optionally the skin treatment dressing is a hydrocolloid dressing.

The skin treatment dressing may be of a light transmitting material. In this respect, the skin treatment dressing may be of a material that allows light from the light emitting part to pass through the material to the skin of a user. The skin treatment dressing may be transparent or translucent.

In embodiments of the invention the skin treatment dressing is for application to a user's skin to occlude a region of the skin being treated.

The skin treatment dressing may be a sheet dressing.

The skin treatment dressing may be configured to support the light emitting part on the user's skin.

Optionally the skin treatment dressing comprises at least one active agent. Active agents are ingredients that work to address a skin condition. The active agent may, for example, be a skin acid such as Salicylic Acid, Vitamin C, or a Peptides or any other active skin care ingredient that has an active effect on the physiology or anatomy of the skin below.

Optionally an outer surface of the skin treatment dressing is provided with a removable layer. This may protect the skin treatment dressing, and keep it sterile, prior to it being applied to a user's skin.

In embodiments of the invention, the skin treatment dressing is attached to the light emitting part. In this respect, optionally the skin treatment dressing is releasably attachable to the light emitting part. This may allow the skin treatment dressing to be replaced after each use. The releasable attachment may be through use of an adhesive, a physical coupling or a magnetic coupling, for example. The skin treatment dressing may be directly attached to the light emitting part. Alternatively, there may be one or more intermediary layers between the light emitting part and the skin treatment dressing.

In embodiments of the invention the light emitting part comprises a housing and a light source. The light source may be provided in the housing. In this case, the housing may have an aperture for passing light from the light emitting part to the skin treatment dressing. Alternatively, the light source may be mounted to an exterior of the housing.

Optionally the housing comprises a first region for releasably attaching the skin treatment dressing to the housing. The first region may have said aperture for passing light from the light emitting part to the skin treatment dressing.

The skin treatment dressing may comprise an adhesive. In this respect, the skin treatment dressing may be of an adhesive material. In this case, the skin treatment dressing may be releasably attachable to the light emitting part due to its adhesive composition/nature. Furthermore, the skin treatment dressing may adhere to a user's skin, in use, due to its adhesive composition/nature.

Alternatively, or additionally, an adhesive may be provided on an upper surface of the skin treatment dressing. In this case, the skin treatment dressing may be releasably attachable to the light emitting part by the adhesive. Alternatively, or additionally, an adhesive may be provided on a lower surface of the skin treatment dressing so that the skin treatment dressing adheres to a user's skin, in use. The adhesive may be a layer of adhesive, for example.

Optionally the dermatologic treatment apparatus comprises a switching apparatus configured to detect the presence of a user's skin and to switch the light emitting part on when the presence of skin is detected. In this respect, the switching apparatus may be configured to detect when the skin treatment dressing is in contact with a user's skin. The switching apparatus may provide for an ease of use, as it automatically switches the light emitting part on, when the presence of skin is detected.

Optionally the switching apparatus comprises first and second electrodes adapted to switch the light emitting part on when the presence of skin is detected by a change in electrical signal between the two electrodes.

Optionally the dermatologic treatment apparatus comprises a timer adapted to control the duration that the light emitting part is on, to deliver a predetermined dose of light to the skin of a user.

Optionally the timer is adapted to activate upon placement of the apparatus onto a user's skin. In this respect, the timer may be configured to activate upon placement of the skin treatment dressing onto a user's skin.

Optionally the dermatologic treatment apparatus comprises a rechargeable battery system configured to power the light emitting part.

Optionally the rechargeable battery system comprises a rechargeable battery and an inductive coupling configured such that the rechargeable battery may be charged by a charger unit by electromagnetic induction.

Optionally the rechargeable battery system comprises a rechargeable battery configured to be charged by a charging attachment when the charging attachment is attached to the apparatus.

According to a second aspect of the invention there is provided a dermatologic treatment apparatus assembly comprising a dermatologic treatment apparatus according to the first aspect of the invention, wherein the rechargeable battery system comprises a rechargeable battery and an inductive coupling configured such that the rechargeable battery may be charged by a charger unit by electromagnetic induction, wherein the dermatologic treatment apparatus assembly also comprises the charger unit.

According to a third aspect of the invention there is provided a dermatologic treatment apparatus assembly comprising a dermatologic treatment apparatus according to the first aspect of the invention, wherein the rechargeable battery system comprises a rechargeable battery configured to be charged by a charging attachment when attached to the apparatus, wherein the dermatologic treatment apparatus assembly also comprises the charging attachment.

Optionally the charging attachment is a charging base.

The skin treatment dressing may be of a shape advantageous to the area of the user that is to be treated. For example the skin treatment dressing may be an under eye tear drop shape, over lip crescent shape, pimple round shape. The skin treatment dressing may be shaped in a manner to allow multiple skin treatment dressings to be located adjacent to each other, for example in a hexagon shape.

Although particular output specifications for light pulses emitted from the light emitting part of the preferred embodiment will be explained in more detail below, an exemplary device in accordance with a preferred embodiment may produce light pulses according to the following summary of parameters:
80mW output power
Spot size 12cm (the patch is a tear drop shape)
Constant Wave (not pulsed)
Wavelength: 633nm

An exemplary device in accordance with a preferred embodiment may produce light pulses according to the following summary of parameters:
Output power: 2.181mW (equating to a 30J/cm over 3 hours)
Spot size (or output aperture area): 0.5 cm2
Pulsewidth: 0.500 s
Pulse repetition frequency: 0.5 Hz
Wavelength: 410 nm

In an embodiment the light emitting part is configured to output light at fluence of between 0.01J/cm2 and 50J/cm2, or a power between 0.1mW and 1000mW, at one or more wavelengths between 300nm and 1600nm. The light source can be pulsed, in a preferred embodiment the light source is pulsed at a repetition rate of up to 0.5 Hz.

In a preferred embodiment the light emitting part outputs a light at 404nm with a fluence of 30J/cm2. This wavelength is absorbed by acne causing c.acne bacteria, the absorbtion of which causes the acne bacteria to self-destruct. In the preferred embodiment, the time of use is 3 hours.

In a second preferred embodiment the light emitting part outputs a light in the red to near infrared spectral range (between 600-1100 nm) with a fluence in the range of 9J/cm2 - 30J/cm2, an output power range of 10mW - 200mW, preferably 80mW per LED, with around 8 LEDs.

These parameters stimulate the production of collagen, elastin, and fibroblasts to enable wound healing within the skin of a user. In the preferred embodiment, the time of use is 3 hours.

In a third preferred embodiment the light emitting part emits multiple bands of light, for example both blue 400nm light and red 600nm light, because the action spectra for tissue regeneration and repair consist of more than one wavelength, it is favourable to apply a polychromatic spectrum covering a broader spectral region for skin rejuvenation and skin repair.

In a fourth preferred embodiment the light emitting part emits multiple bands of light, for example both red 633nm and infra red 1072nm light.

Optionally the skin treatment dressing comprises at least one light transmitting region that is configured to provide for greater transmission of light, from the light emitting part to the skin of a user, than another region of the skin treatment dressing.

A problem with the use of a skin treatment dressing (for example a hydrocolloid), is that the material has a degree of light absorption and this reduces the efficiency of the light therapy. In tests the inventors found that this absorption was dependent on the nature of the material, the wavelength of the transmitted light (the absorption of light is directly proportional to the frequency) and the thickness of the skin treatment dressing (the absorption of light is directly proportional to the thickness).

The obvious solution to this problem for a given material is to increase the light output of the light emitting part to compensate for the loss or decrease the thickness of the light transmitting layer. However, both of these solutions provide additional difficulties.

Increasing the light output requires a greater amount of power, and therefore increases the power source size or, if the power source is not increased, reduces the maximum therapeutic treatment time. The apparatus is required to be affixed to the skin, and therefore a greater sized power source (which is typically larger and heavier) makes any affixation difficult to achieve and uncomfortable for a user (the weight of the powersource is held by the skin). Reducing maximum therapeutic treatment time is also unacceptable - typically to treat a skin condition with phototherapy a certain light dose is required. The inventors found in tests that with commonly available batteries that increasing the light output to the level required to treat a skin condition resulted in a therapeutic treatment time less than that required to provide a therapeutic dose. In addition, the inventors found that increasing the light output also resulted in an proportional increase of heat in the skin treatment dressing. This resulted in the skin treatment dressing becoming overly compilable, losing its adhesive properties and failing to affix to the skin.

Likewise, it was found that reducing the thickness of the skin treatment dressing resulted in the material being too thin to provide enough support to allow the skin treatment dressing and light emitting part to remain affixed to the skin.

The at least one light transmitting region allows for greater transmission of light from the light emitting part, through the skin treatment dressing, to the user's skin, thereby increasing the efficiency of the light therapy.

Furthermore, these advantages may be provided whilst allowing the skin treatment dressing to still be able to provide enough support to allow the skin treatment dressing and light emitting part to remain affixed to the skin. In this respect, it avoids having to provide increased power and therefore avoids having to use a larger power source, thereby making easier to affix to skin of user and making more comfortable to wear for the user. Also, this can provide the required therapeutic time without having to increase the power of the power source. Also, since it is not needed to increase the power, this avoids an increase in heat in the skin treatment dressing. Accordingly, this avoids the skin treatment dressing becoming overly compliable and so it doesn't lose its adhesive properties and thus doesn't fail to affix to the user's skin.

The light emitted from the light emitting part may have one or more wavelengths between 300nm and 1600nm, preferably the light shall have wavelengths between 400nm and 1100nm and even more preferably at two or more wavelengths peaks between 400nm and 420nm, 630nm and 690nm and 1010nm and 1080nm respectively. The light emitting part (for example, the light emitting source, see below) may output each of these wavelength peaks instantaneously, or may output each in a sequence of one or more wavelengths, which may be under the control of the control module (see below). For example, the light emitting part (e.g. under the control of the control module) may emit a first light at 400-420nm for a first predetermined duration, followed by a second light of 630nm-690nm and 101nm-1080nm for a second predetermined duration. Such a sequence may be beneficial for certain physiological skin conditions such as acne vulgaris and psoriasis where the sequential nature of the treatment provides an improved treatment of the condition compared to non sequential treatment. This is particularly apparent, where the use of 400nm-420nm light is required for a typically lower duration than 630nm-690nm and where over exposure of 400-420nm light can result in complications in treatment such as hyperpigmentation. The light may be emitted continuously or pulsed, for example under the control of the control module (see below).

The light emitted from the light emitting part may be red light. It may have a wavelength of 585 to 830, perferably 633 nm.

The light emitted from the light emitting part may be blue light. It may have a wavelength of wavelength of 407-420 nm).

In embodiments of the invention the light emitting part comprises a light source and the apparatus comprises a power source configured to power the light source. Optionally the apparatus comprises a control module configured to control the powering of the light source by the power source.

The light source may comprise one or more individual light sources and may consist of LEDs including organic, inorganic and Quantum Dot LEDs, electroluminescent material or any other light source capable of emitting a light suitable for causing a therapeutic effect within the skin and body of a user.

The one or more light sources may be selected with parameters suitable causing a therapeutic effect within the skin or body of a user. In this respect, the one or more light sources may output light with said wavelength(s).

Optionally the control module comprises hardware for controlling the light source, such as a microcontroller. The control module may comprise a user interaction part to allow a user to control the output of the light source. Optionally the apparatus is configured such that the user may control the output based upon a desired result for a specific skin condition and may select a 'therapy mode' with predetermined light output characteristics for which the control module will control the light source. Alternatively the apparatus may be configured such the user may select the light output directly and control which output parameters (for example wavelength, duration, intensity) are chosen. The control module may further comprise a communication part for wireless communication with a third apparatus, for example a bluetooth or infrared connection to a smartphone, to allow remote control of the apparatus. The apparatus may be configured such that a user may direct control of the apparatus through a downloaded SmartPhone App or by a wireless connection to a remote computer.

Optionally the light emitting part comprises a rigid, or semi rigid, casing. The light source may be provided in the casing. Alternatively, or additionally, the light source may be affixed to the exterior surface of the casing. The power source and control module may be housed within the casing.

The power source may comprise a battery and battery control hardware. The battery may be rechargeable via a second power source, for example a mains electric, and the power source may further comprise charging hardware adapted to allow for the charging of the power source. The charging hardware may comprise an electrical charging socket or may allow for wireless charging through use of an inductive charging apparatus.

Optionally the skin treatment dressing is flexible. Optionally the skin treatment dressing has an adhesive skin contacting front face and a rear face adapted to affix to the light emitting part. The flexible skin treatment dressing may have an adhesive nature. The rear face may affix to the light emitting part by means of the adhesive nature of the flexible skin treatment dressing. Alternatively, or additionally, the sin treatment dressing may be coupled to light emitting part by a mechanical or magnetic coupling.

Optionally the flexible skin treatment dressing is a hydrocolloid. However other materials such as a hydrogel could be used. A hydrocolloid is preferred over a hydrogel due to its inherent occlusive and adhesive properties which can aid phototherapy treatment and its ability to further contain ingredients to support therapeutic treatment, for example Salicylic Acid can be incorporated into the hydrocolloid material to assist in the treatment of acne vulgaris.

Optionally the at least one light transmitting region has a substantially circular cross-sectional shape.

Optionally the circular cross-sectional shape has a diameter between 0.01mm and 1mm.

Optionally the circular cross-sectional shape has a diameter between 0.1mm and 0.5mm. The shape may be chosen for the therapeutic region being treated for example under eye or over lip have a tear drop and arc shape.

Optionally the at least one light transmitting region is a light transmitting channel.

Optionally the at least one light transmitting region is configured to allow the transmission of light from the light emitting part to the skin of the user without attenuation of the light.

Optionally the light transmitting channel extends along a longitudinal axis that is perpendicular to the face of the skin treatment dressing.

Optionally the at least one light transmitting region is a region of reduced thickness of the skin treatment dressing.

Optionally the skin treatment dressing is a single layer of skin treatment dressing that has at least one region of reduced thickness to provide said light transmitting region.

Optionally the skin treatment dressing comprises a plurality of layers of skin treatment dressing arranged to provide said at least one region of reduced thickness of the skin treatment dressing.

Optionally the skin treatment dressing comprises a first layer of skin treatment dressing that has a substantially uniform thickness and a second layer of skin treatment dressing that has at least one channel or region of reduced thickness such that the combination of the first and second layers has said at least one light transmitting region.

Optionally the skin treatment dressing comprises first and second layers of skin treatment dressing that each have at least one channel or region of reduced thickness, and wherein the at least one channel or region of reduced thickness in the first and second layers are at least partially offset from each other such that the combination of the first and second layers has said at least one light transmitting region.

Optionally the skin treatment dressing comprises a plurality of said light transmitting regions.

Optionally the light transmitting regions are clustered into a plurality of arrays.

Optionally the plurality of arrays are evenly distributed across the surface of the skin treatment dressing.

Optionally the light emitting part comprises a plurality of light sources and wherein the arrays are each clustered around a respective light source.

Optionally the light transmitting regions are distributed in a hexagonal configuration.

Optionally the light transmitting regions are distributed in a hexagonal configuration at intervals between 0.05mm and 2mm apart.

Optionally said intervals are between 0.5mm and 1mm apart.

According to a fourth aspect of the invention there is provided a method of dermatological treatment comprising providing a dermatologic treatment apparatus comprising:
a light emitting part, for emitting light for dermatological treatment;
wherein the apparatus further comprises a skin treatment dressing,
the skin treatment dressing is a bioactive dressing;
the skin treatment dressing is arranged to adhere to a user's skin, during use;
and wherein the skin treatment dressing is configured such that the light from the light emitting part passes through the skin treatment dressing to a user's skin;
wherein the method comprises:
   applying the skin treatment dressing to a skin surface to be treated and transmitting light from the light emitting part through the skin treatment dressing to the skin surface.

Optionally the skin treatment dressing is arranged with the light emitting part such that, in use, light from the light emitting part passes to the skin treatment dressing.

The skin treatment dressing may be applied to the skin surface to at least partially cover a skin condition. The skin treatment dressing may be applied to the skin surface to cover a skin condition.

The skin condition may, for example, be acne, rosacea, wrinkles, dark spots, hyperpigmentation, hypopigmentation, scaring or cellulite or tissue damage, wounding, cold sores, abscess, ulcers, boils, dermatitis, alopecia, cheilitis, bites or stings, tinea pedis, warts, milia, cradle cap, eczema, erythrasma, folliculitis, fungal or bacterial infections such as onychomycosis, lentigo, or other medical or dermatological conditions or an age related skin conditions such as wrinkles, rhytides, periorbital puffiness, periocular hyperpigmentation, melanosis.

Optionally the skin treatment dressing is applied to the skin surface, and the light is transmitted from the light emitting part, through the skin treatment dressing to the skin surface, for a period of at least one minute. The period may be for at least 10 minutes. The period may be of at least 1 hour. The period may be between 1 minute and 1 hour. The period may be of at least 6 hours. The period may be of at least 3 days.

Optionally an outer surface of the skin treatment dressing is provided with a removable layer and wherein the removeable layer is removed prior to applying the skin treatment dressing to the skin surface.

After the light emitting part has emitted light for a certain period of time, to a user's skin, the light emitting part may be operated to stop emitting light to the user's skin, whilst the skin treatment dressing remains attached to the user's skin for a further period of time. For example, the light emitting part may be detached from the skin treatment dressing to stop emitting light to the user's skin. Alternatively, or additionally, the light emitting part may be switched off.

For example, the light emitting part may emit light for 10 minutes, but the light emitting part then removed from the skin treatment dressing, with the skin treatment dressing remaining attached to the skin for 3 hours.

According to a fifth aspect of the invention there is provided method of manufacture of a dermatologic treatment apparatus, comprising:
providing a light emitting part, for emitting light for dermatological treatment;
providing a skin treatment dressing,
wherein the skin treatment dressing is a bioactive dressing;
and the skin treatment dressing is arranged to adhere to a user's skin, during use;
arranging the skin treatment dressing with the light emitting part such that, in use, light from the light emitting part passes to the skin treatment dressing;
and configuring the skin treatment dressing such that the light from the light emitting part passes through the skin treatment dressing to a user's skin.

Optionally the skin treatment dressing is cut from a length of skin treatment dressing.

Optionally the method of manufacture is repeated, so as to manufacture a plurality of dermatologic treatment apparatus and wherein each skin treatment dressing is cut from the same length of a skin treatment dressing. This may provide for efficient and quick manufacture of multiple dermatologic treatment apparatus.

According to a sixth aspect of the invention there is provided a kit of parts comprising:
a light emitting part, for emitting light for dermatological treatment;
a skin treatment dressing,
wherein the skin treatment dressing is a bioactive dressing;
the skin treatment dressing being arranged to adhere to a user's skin, during use;
the skin treatment dressing is for being arranged with the light emitting part such that, in use, light from the light emitting part passes to the skin treatment dressing;
and wherein the skin treatment dressing is for being configured such that the light from the light emitting part passes through the skin treatment dressing to a user's skin.

The features of any of the above aspects of the invention may be combined with one or more features of any of the other aspects of the invention, in any combination. In this respect, features of any apparatus claim may be combined with features of any method claim, in any combination, and vice-versa.

Other preferred and advantageous features of the invention will be apparent from the following description.

### Description of the Drawings

A specific embodiment of the invention will now be described with reference to the description and drawings.
Figure 1 shows a cross-sectional view of a dermatologic treatment apparatus according to a first embodiment of the invention (where the skin treatment dressing is shown spaced slightly below the light emitting part, for illustrative purposes);
Figure 2 shows a view corresponding to that of Figure 1, but where the dermatologic treatment apparatus is according to a second embodiment of the invention;
Figure 3 shows a view from below of a dermatologic treatment apparatus according to a third embodiment of the invention;
Figure 4 shows an electrical light circuit of the dermatologic treatment apparatus of Figure 3;
Figure 5 shows a charging circuit of a dermatologic treatment apparatus according to a fourth embodiment of the invention;
Figure 6 shows a schematic side view of a dermatologic treatment apparatus according to a fifth embodiment of the invention;
Figure 7 shows the skin treatment dressing of the apparatus in Figure 6;
Figure 8 shows a cross-section view of the skin treatment dressing of a dermatologic treatment apparatus according to a sixth embodiment of the invention;
Figure 9 shows a cross-section view of the skin treatment dressing of a dermatologic treatment apparatus according to seventh embodiment of the invention;
Figure 10 shows a cross-section view of the skin treatment dressing of a dermatologic treatment apparatus according to an eighth embodiment of the invention;
Figure 11a shows a view from below of a dermatologic treatment apparatus according to a ninth embodiment of the invention (where the skin treatment dressing is not attached to the light emitting part of the apparatus);
Figure 11b shows a view from above of the dermatologic treatment apparatus in Figure 11a;
Figure 11c shows an exploded perspective view of the dermatologic treatment apparatus in Figures 11a and 11b (along with a charging cable);
Figures 12a to 12d show examples of the arrangement /pattern of the light transmitting regions of the skin treatment dressing of the dermatologic treatment apparatus in Figures 11a to 11c.

### Detailed Description

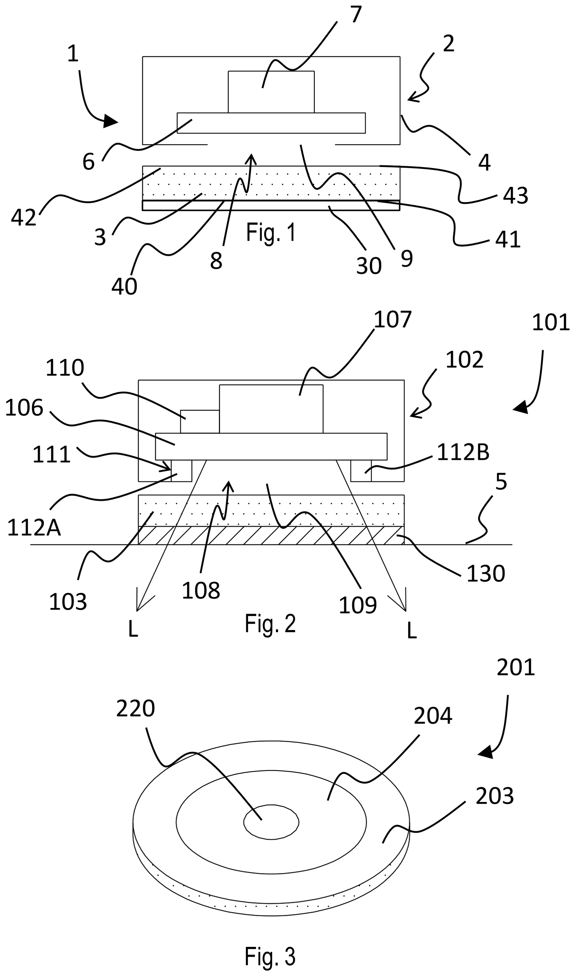

Referring to Figure 1 there is shown a skincare apparatus, in the form of a dermatologic treatment apparatus 1 comprising a light emitting part 2 and a skin treatment dressing 3 releasably attached to the light emitting part 2.

The light emitting part 2 comprises a self-contained housing 4 configured for adhering to the skin 5 of user for a dermatologic treatment procedure. A light source 6, which may include one or more light emitting diodes and/or diode lasers, is mounted in the housing 4. The light emitting diodes may be inorganic or organic LEDs such as quantum dot LEDs. The light source 6 and an associated electrical circuit are contained within the housing 4. The electrical circuit includes one or more power sources 7 for energizing the light source 6 to produce output light pulses sufficient for providing efficacious dermatological treatment.

In the above described embodiment the power source 7 is a 3.7V 24mAh printed lithium battery. This battery can power multiple LEDs and has an ultrathin profile allowing for the housing to have a low profile (the higher the device is the more it pivots on the skin which is uncomfortable for a user as it tugs on the skin)

The power source 7 may, for example, be one or more small battery cells rated at 1.2 V each and providing between 1.0 V and 1.5 V during the course of a discharge period. A typical LED uses about 20mA and the capacity of a CR2032 Coin Cell is 200mAh. This allows the skincare apparatus using a single LED light source 10 hours of use, or 3 applications of the skincare apparatus before replacement.

The housing 4 further comprises a first region 8 for releasably attaching the skin treatment dressing 3.

A light path (L) within the housing includes an aperture 9 within the first region 8 through which the output light is propagated out of the housing 4 and through the skin treatment dressing 3.

In this respect, the skin treatment dressing 3 is of a material that allows light from the light emitting part 2 to pass through the material to the skin 5 of a user. The skin treatment dressing 3 is transparent or translucent.

The skin treatment dressing 3 is a bioactive dressing. In the currently described embodiment, the skin treatment dressing is a hydrocolloid dressing. Hydrocolloids are a heterogeneous group of long chain polymers (polysaccharides and proteins) characterised by their property of forming viscous dispersions and/or gels when dispersed in water. They are transparent or translucent.

The skin treatment dressing 3 is for application to a user's skin to occlude a region of the skin being treated. Furthermore, the skin treatment dressing 3 comprises one or more active agents. In exemplary embodiments, the active agent is salicylic acid, benzoyl peroxide, retinoid, alpha hydroxy acid, poly hydroxy acid, hyaluronic acid or sulphur, a retinol or retinol esther, peptide, Sodium Hyaluronate, Niacinamide, Caffeine, Camellia Sinensis Leaf Extract (Green Tea), Azelaic acid or Ascorbic Acid. An example of peptide compounds could be Matrixyl 3000 and/or Eyeliss, both being trademarked peptide compositions developed by Sederma Inc.

In an exemplary embodiment, the active ingredients are Niacinamide, Caffeine, Eyeliss, Matrixyl 3000, Sodium Hyaluronate and Sodium hydroxide.

In a further exemplary embodiment, the active ingredients are Sodium Hyaluronate, Camellia Sinensis Leaf Extract (Green Tea), Salicylic Acid, Niacinamide, Oligopeptide-76 and Sodium hydroxide.

In a further exemplary embodiment, the active ingredients are Azelaic acid, Ascorbic Acid, Oligopeptide-34, Sodium Hyaluronate, Sodium hydroxide.

The skin treatment dressing may be any type of Bioactive dressing. Bioactive dressings are dressings that deliver substances active in wound healing, by delivery of bioactive compounds or constructed from materials having endogenous activity. These materials include hydrocolloids, alginates, collagens, chitosan, chitin, derivatives from chitosan or chitin and biotextiles.

The skin treatment dressing 3 comprises one or more active agents selected from the group consisting of salicylic acid, benzoyl peroxide and sulphur associated with the hydrocolloid dressing, wherein the active agent is present at an amount so as to not reduce the absorption capability of the hydrocolloid dressing.

In exemplary embodiments, the active agent is present at an amount of about 0.01% to about 10%, for example, the active agent is salicylic acid present at an amount of about 0.1% to about 2%, or about 0.5% to about 1.2%. In additional embodiments, the active agent is benzoyl peroxide present at an amount of about 2.5% to about 10%, or in further embodiments, the active agent is sulfur present at an amount of about 3% to about 10%.

Also provided herein is an apparatus for treatment of acne, consisting essentially of a hydrocolloid dressing, and salicylic acid present at an amount of about 0.5% to about 1.5%.

In further embodiments, the hydrocolloid dressing is in the form of an application pad having a size of about 0.5 cm to about 4 cm.

The skin treatment dressing 3 is a sheet dressing.

The skin treatment dressing 3 is configured to support the light emitting part 2 on the user's skin.

The skin treatment dressing 3 is releasably attachable to the light emitting part 2. This allows the skin treatment dressing 3 to be replaced after each use. In the currently described embodiment the skin treatment dressing 3 has a rear face 42 that is provided with a layer of adhesive 43 so that the skin treatment dressing 3 is releasably adhered to the lower surface of the housing 4. However, it will be appreciated that any suitable form of releasable attachment may be used, for example a physical connector such as hook and loop or a magnetic connection. Alternatively, or additionally, the skin treatment dressing 3 may of an adhesive material, so that the skin treatment dressing 3 itself releasably adheres to the lower surface of the housing 4.

The skin treatment dressing 3 has skin contacting front face 40 that is provided with a layer of adhesive 41 so as to adhere to a user's skin in use. In the currently described embodiment, the adhesive is a glue. In the currently described embodiment, the adhesive is a standard tissue adhesive. It will be appreciated that any suitable adhesive may be used, including a polyethylene film or a silicone adhesive, for example.

Alternatively, or additionally, the skin treatment dressing may of an adhesive material, so that the skin treatment dressing itself adheres to the skin of a user in use. This advantageously allows the dermatologic treatment apparatus 1 to adhere to the skin of a user during use, thereby helping the keep the apparatus in place over the region of the skin to be treated.

The skin contacting front face 40 is provided with a removable layer 30. This protects the skin treatment dressing 3, and keep it sterile, and also protects the adhesive 41, prior to it being applied to a user's skin.

The skin treatment dressing 3 is configured such that, in use, light from the light emitting part 2 passes through the skin treatment dressing 3 to the skin of a user. In this respect, the skin treatment dressing 3 is of a material that is transparent to the light emitted from the light emitting part 2.

In the described embodiment, the light emitting part emits light pulses according to the following summary of parameters:
Output power: 2.181mW (equating to a 30J/cm over 3 hours)
Spot size (or output aperture area): 0.5 cm2
Pulsewidth: 0.500 s
Pulse repetition frequency: 0.5 Hz
Wavelength: 410 nm

In alternative embodiments (i.e. for each of the described embodiments), the light source outputs a light at 404nm with a fluence of 30J/cm2. This wavelength is absorbed by acne causing c.acne bacteria, the absorbtion of which causes the acne bacteria to self-destruct. In the preferred embodiment, there is 10 minutes of phototherapy and 3 hours of the hydrocolloid dressing remaining in place.

In further alternative embodiments (i.e. for each of the above described embodiments), the light source outputs a light in the red to near infrared spectral range (between 600-1100 nm) with a fluence of 9J/cm2 to 20J/cm2, preferably 17J/cm2. These parameters are known to stimulate the production of collagen, elastin, and fibroblasts to enable wound healing within the skin of a user. In the preferred embodiment, there is 10 minutes of phototherapy and 3 hours of the hydrocolloid dressing remaining in place.

In yet further alternative embodiments (i.e. for each of the above described embodiments), the light source outputs multiple bands of light, for example both blue 400nm light and red 600nm light, or 633nm and 1072nm light because the action spectra for tissue regeneration and repair consist of more than one wavelength, it is favourable to apply a polychromatic spectrum covering a broader spectral region for skin rejuvenation and skin repair.

The skin treatment dressing 3 occludes the region undergoing treatment, thereby preventing the user picking or otherwise interfering with a skin condition which might otherwise slow down the healing process, whilst allowing light, from the light emitting part 2, to pass through it to provide dermatological treatment. Accordingly the apparatus 1 provides the advantages of both a skin treatment dressing and of phototherapy. In this respect, the apparatus 1 provides the long term benefits and ease of use of a skin treatment dressing with the immediate results of phototherapy.

Referring to Figure 2, there is shown a dermatologic treatment apparatus 101 according to a second embodiment of the invention. The dermatologic treatment apparatus 101 of the second embodiment is the same as that of the first embodiment, except for the differences described below. Corresponding features are given corresponding reference numerals, but incremented by 100.

In the second embodiment, the dermatologic treatment apparatus 101 further comprises a switching apparatus 111 for activating the light source 106. In the preferred embodiment the switching apparatus 111 is configured to detect the presence of skin 5 using an electrical signal between two electrodes 112A, 112B, located on either side of the first region 108 and adapted to activate the electrical circuit when the presence of skin 5 is detected by a change in the electrical signal. Other switching apparatus could be used including mechanical switches, timed switches, and wireless switches, or a combination thereof without departing from the scope of the invention.

The treatment apparatus 101 further comprises a timer 110, said timer 110 adapted to deliver a predetermined dose of light to the skin 5 of a user, and further adapted to activate upon placement of the skincare apparatus to a user's skin. In this respect, the timer 110 and switching apparatus 111 are electrically configured to activate simultaneously upon skin contact.

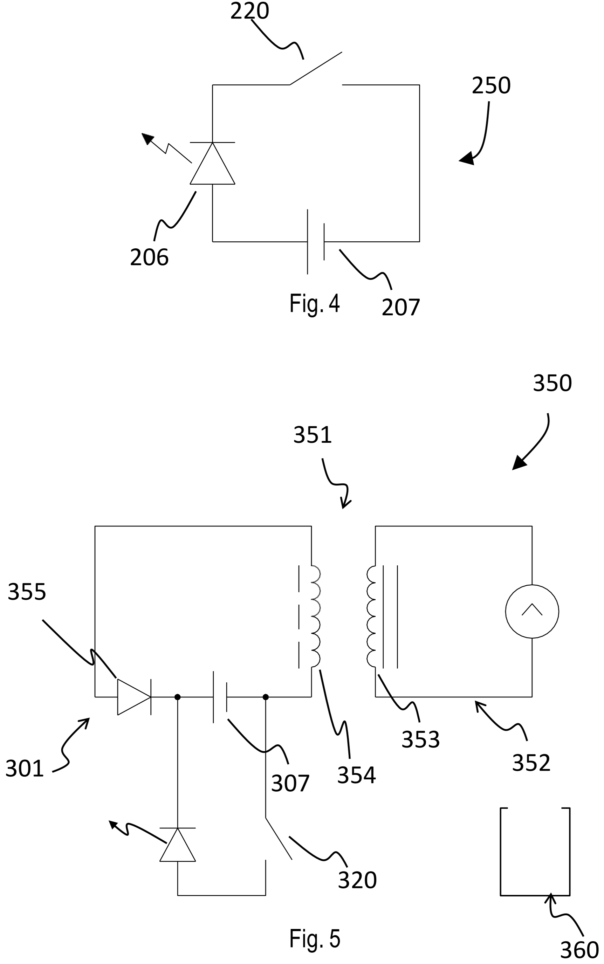

Referring to Figure 3, there is shown a dermatologic treatment apparatus 201 according to a third embodiment of the invention. The dermatologic treatment apparatus 201 of the third embodiment is the same as that of the first embodiment, except for the differences described below. Corresponding features are given corresponding reference numerals, but incremented by 200. Figure 4 shows the electrical circuit 250 of the dermatologic treatment apparatus 201 of the third embodiment.

The dermatologic treatment apparatus 201 of the third embodiment comprises a mechanical switch 220 that is pushed when the treatment apparatus 201 is placed onto a user's skin.

This closes the switch 220 (see Figure 4), which causes the power source 207 to power the light source 206.

Referring to Figure 5, there is shown the electrical circuit of a dermatologic treatment apparatus 301 according to a fourth embodiment of the invention. The dermatologic treatment apparatus 301 of the fourth embodiment is the same as that of the third embodiment, except for the differences described below. Corresponding features are given corresponding reference numerals, but incremented by 100.

In the dermatologic treatment apparatus 301 of the fourth embodiment the electrical circuit 350 further comprises a rechargeable battery system 351 in which the apparatus regulates a transfer of energy from a separate charger unit 352. For recharging, a charger unit 352 is brought into proximity to the apparatus 301. An oscillating current is generated in a primary coil 353, located in the charger unit 352. By inductive coupling through an oscillating magnetic field, an alternating current is generated in a secondary coil 354 within the electrical circuit 350. The alternating current then passes through a half-wave or full-wave rectifier 355 to form a one-sided current, then passes through a regulator (not shown) to form a direct current, which is in turn directed to the rechargeable battery 307 in the electrical circuit.

In a preferred embodiment, between uses of the skincare apparatus is preferably placed in a charging base 360. The charging base 360 may be similar to those currently produced for use with electric toothbrushes, shavers, phones, etc. The base 360 is connected to a standard AC outlet, and is capable of recharging the batteries overnight. In a further preferred embodiment, the charging base comprises a casing having a shallow tray positioned above a primary coil electrically connected to a charging circuit and a mains electric supply. In use one or more skincare apparatus are positioned in the shallow tray to charge.

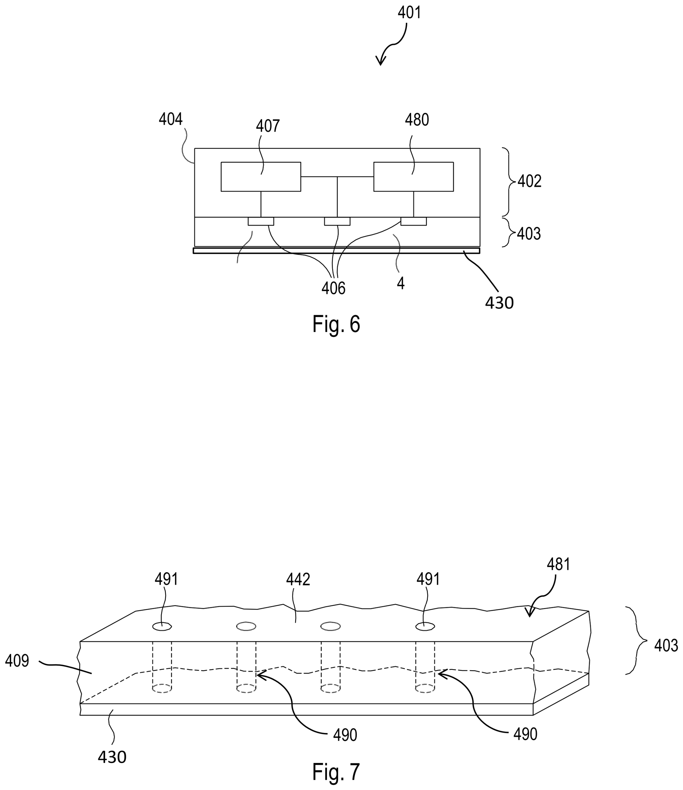

Referring to Figures 6 and 7 there is shown a dermatologic treatment apparatus 401 according to a fifth embodiment of the invention. The dermatologic treatment apparatus 401 of the fifth embodiment is the same as that of the first embodiment, except for the differences described below. Corresponding features are given corresponding reference numerals, but incremented by 400.

The apparatus 401 comprises a light emitting part 402 and a skin treatment dressing 403.

The light emitting part 402 comprises a rigid, or semi rigid, housing 404. A light source 406 is affixed to the exterior surface of the housing 404. A power source 407 and a control module 480 are housed within the housing 404. The control module 480 is electrically interconnected to the light source 406 and power source 407 and adapted to control the powering of the light source 406 by the power source 407.

The control module 480 comprises hardware for controlling the light source of the light emitting part, such as a microcontroller. The control module 480 comprises a user interaction part to allow a user to control the output of the light source. The apparatus is configured such that the user may control the output based upon a desired result for a specific skin condition and may select a 'therapy mode' with predetermined light output characteristics for which the control module will control the light source. Alternatively the apparatus may be configured such the user may select the light output directly and control which output parameters (for example wavelength, duration, intensity) are chosen. The control module may further comprise a communication part for wireless communication with a third apparatus, for example a bluetooth or infrared connection to a smartphone, to allow remote control of the apparatus. The apparatus may be configured such that a user may direct control of the apparatus through a downloaded SmartPhone App or by a wireless connection to a remote computer.

The power source 407 comprises a battery and battery control hardware (not shown). The battery may be rechargeable via a second power source, for example a mains electric, and the power source may further comprise charging hardware adapted to allow for the charging of the power source. The charging hardware may comprise an electrical charging socket or may allow for wireless charging through use of an inductive charging apparatus. Such a charging apparatus is well known in the art.

The skin treatment dressing 403 is a bioactive skin treatment dressing in the form of a hydrocolloid dressing, which is of a flexible light transmitting material 481 (see Figure 7).

The light transmitting material 481 comprises one or more light transmitting regions 490 in the form of channels 490. The channels 490 are circular in cross section and between 0.1mm and 0.5mm in diameter.

The light transmitting regions 490 comprise apertures 491 perpendicular to the rear face of the skin treatment dressing 403 to allow the transmission of light from the light emitting part 402 to the skin of a user without attenuation by the light transmitting material 481 of the skin treatment dressing 403.

In this respect, the light transmitting regions 490 are configured to provide for greater transmission of light, from the light emitting part 402 to the skin of a user, than the other regions of the light transmitting material 481 (i.e. the regions that are not said light transmitting regions 490).

Accordingly, the light transmitting regions 490 increase the efficiency of the light therapy.

Furthermore, this increase in efficiency may be provided whilst allowing the skin treatment dressing 403 to still be able to provide enough support to allow the apparatus remain affixed to the skin. In this respect, it avoids having to provide increased power and therefore avoids having to use a larger power source, thereby making easier to affix to skin of user and making more comfortable to wear for the user. Also, this can provide the required therapeutic time without having to increase the power of the power source. In addition, since it is not needed to increase the power, this avoids an increase in heat in the light transmitting material 490. Accordingly, this avoids the light transmitting material becoming overly compliable and so it doesn't lose its adhesive properties and thus doesn't fail to affix to the user's skin.

The light transmitting material 481 is physically strong enough to affix the light emitting part 402 to the user's skin and maintain its structural integrity when being handled by a user. The inventors have found in tests that when apertures were formed to allow for light transmission, this structural integrity requirement may limit the minimum cross sectional thickness of the light transmitting material. The minimum cross sectional thickness is dependent on the light transmitting material properties. A high minimum cross sectional thickness results in an increase in light absorption by the light transmitting material in areas where there are no light transmitting regions, and a resultant drop in the light dose delivered to the skin.

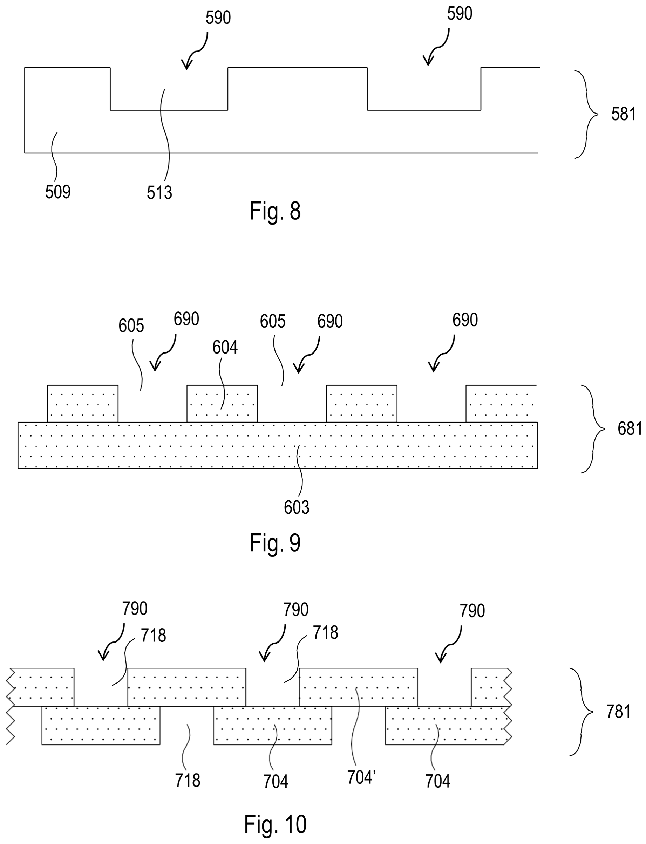

To overcome this problem, in a sixth embodiment, shown in Figure 8, the light transmitting regions 590 are areas of light transmitting material 581 with less cross sectional thickness 513.

The sixth to eighth embodiments are the same as the fifth embodiment, except for the differences described in relation to that embodiment. Corresponding features are given corresponding reference numerals, but incremented by 100 relative to the previous respective embodiment.

Referring to Figure 8, the light transmitting material 581 of the skin treatment dressing 503 comprises a single homogeneous sheet of light transmitting material 581 molded or otherwise produced to have regions of decreased thickness 513 that form the light transmitting regions 590.

Referring to Figure 9, in a seventh embodiment the light transmitting material 681 comprises two layers, with a continuous first layer 603 upon which a second layer 604 with apertures 605 is affixed, to provide said light transmitting regions 690.

Referring to Figure 10, in an eighth embodiment, the light transmitting material 781 comprises two layers combined 704, 704' each layer having apertures 718 therein, and wherein the combined layers are configured such that each aperture 718 is offset, to provide said light transmitting regions 790.

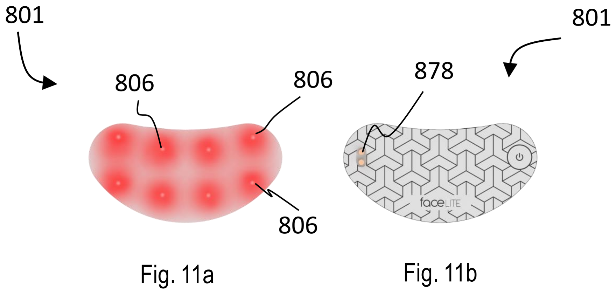

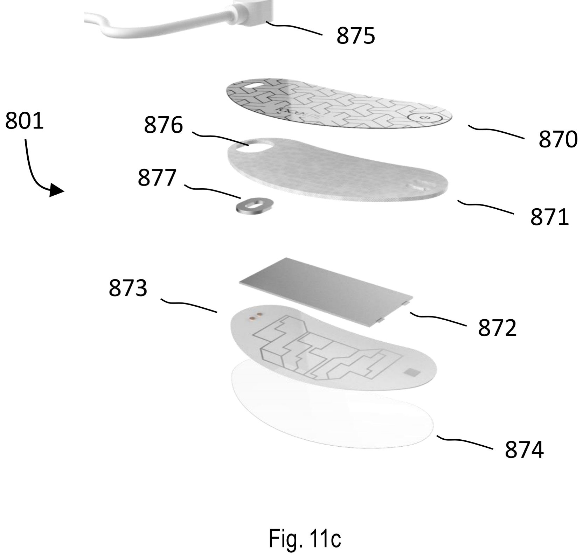

Referring to Figures 11a to 11c, there is shown a dermatologic treatment apparatus 801 according to a ninth embodiment of the invention. The dermatologic treatment apparatus 801 of the ninth embodiment is the same as that of the first embodiment, except for the differences described below. Corresponding features are given corresponding reference numerals, but incremented by 800.

The dermatologic treatment apparatus 801 of the ninth embodiment comprises a plastic upper layer 870 and a flexible gasket layer 871 attached to a lower surface of the plastic upper layer 870. The gasket layer 871 is provided with an aperture 876 in which an annular iron plate 877 is mounted. The annular iron plate 877 is for charging absorption.

A thin battery 872 is provided underneath the gasket layer 871, which is electrically connected to a printed circuit board 873 mounted underneath the battery 872. The light source 806, which are LEDs 806, and control unit are provided on the printed circuit board 873. A plastic sealing layer 874 is provided underneath the printed circuit board 873. The skin treatment dressing (not shown) is releasably adhered to the undersurface of the plastic sealing layer 874, as in the previously described embodiments.

Each of the plastic upper layer 870, gasket layer 871 and printed circuit board 873 have a general tear drop shape so that the overall apparatus 801 has a general tear drop shape. This may be advantageous to the area of the user that is to be treated. Other advantageous shapes may be used, for example an over lip crescent shape, pimple round shape. The skin treatment dressing may be shaped in a manner to allow multiple skin treatment dressings to be located adjacent to each other, for example in a hexagon shape.

An electrical connector 878, to connect the batter 872 to to a power cable 875, for connection to a power supply, is provided in the plastic upper layer 870.

The overall apparatus 801 is flexible and so can be worn comfortably by a user. The overall apparatus 801 is also thin. In this respect, it is less than 2mm thick.

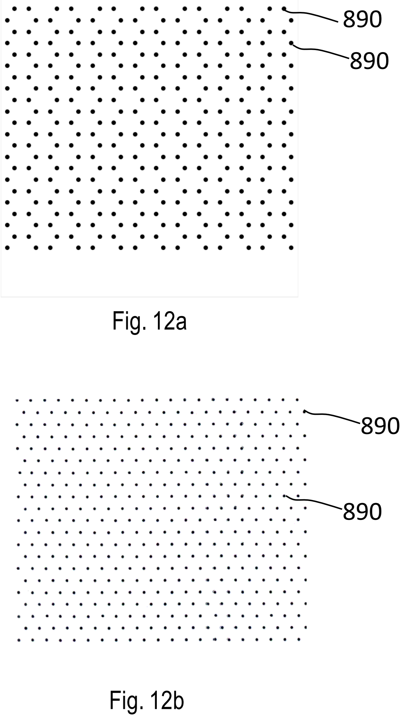

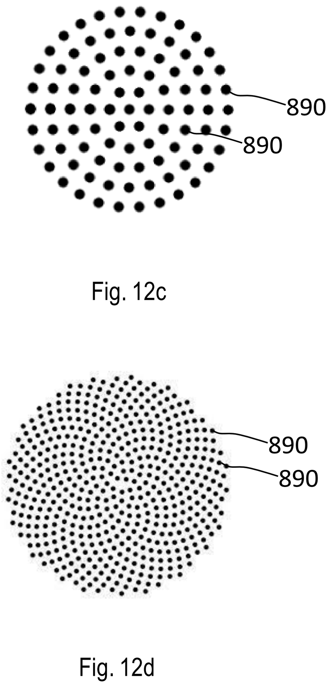

Examples of the arrangement/pattern of the light transmitting regions 890 is shown in Figures 12a to 12d. The light transmitting regions 890 are clustered into a plurality of arrays. In Figure 12b the plurality of arrays are evenly distributed across the surface of the skin treatment dressing. In Figure 12c the arrays are each clustered around a respective light source. In Figure 12a the light transmitting regions are distributed in a hexagonal configuration, at interval that are between 0.5mm and 1mm apart. In a further embodiment, the intervals may be between 0.05mm and 2mm apart.

Any of the described arrangements of light transmitting regions may be used in any of the described embodiments.

In the described embodiments, the skin treatment dressing comprises a removable layer covering the inner surface of the dressing. In additional embodiments, at least two of the dressings are attached to the same removable layer. Suitably, the plurality of dressings have square, circle or oval shapes. The plurality of dressings have a shape suitable for the region of application on a users skin - for example square, circle, oval, tear drop or shaped in a manner enabling two or more dressings to be aligned adjacent to each other.

As described above, the skin treatment dressing is configured such that, in use, light from the light emitting part passes through the skin treatment dressing to the skin of a user. In this respect, the skin treatment dressing may occlude the region undergoing treatment, thereby preventing the user picking or otherwise interfering with a skin condition which might otherwise slow down the healing process, whilst allowing light, from the light emitting part, to pass through it to provide dermatological treatment. Accordingly the apparatus may provide the advantages of both a skin treatment dressing and of phototherapy. In this respect, the apparatus may provide the long term benefits and ease of use of a skin treatment dressing with the immediate results of phototherapy.

According to a further embodiment, a method of treatment comprises provide a said apparatus, applying the apparatus to a skin surface, the apparatus at least partially covering the skin condition, and 10 minutes of phototherapy and 3 hours of the hydrocolloid dressing remaining in place, thereby treating the skin condition. In suitable embodiments, the condition is either acne, rosacea, wrinkles, dark spots, hyperpigmentation, hypopigmentation, scaring or cellulite.

Suitably, the apparatus is maintained on the skin surface for a period of at least 6 hours, and in exemplary embodiments, the method of treatment is repeated daily for a period of at least 3 days.

In the second embodiment, the apparatus further comprises a removable layer 130 on an outer surface of the hydrocolloid dressing 3, and the method further comprises removing the removable layer 130 prior to applying the apparatus to the skin surface 5.

In suitable embodiments, the amount of salicylic acid is about 0.1% to about 1.5%.

The methods can also further comprise cutting the hydrocolloid dressing into a plurality of separate devices.

In a further embodiment, a method of dermatological treatment comprises providing a dermatologic treatment apparatus according to any of the described embodiments, wherein the method comprises applying the skin treatment dressing to a skin surface 5 to be treated and transmitting light from the light emitting part through the skin treatment dressing to the skin surface 5.

The skin treatment dressing is applied to the skin surface 5, and the light is transmitted from the light emitting part through the skin treatment dressing to the skin surface 5, for a period of at least one hour.

Where an outer surface of the skin treatment dressing is provided with a removable layer this is removed prior to applying the skin treatment dressing to the skin surface 5. This protects the skin treatment dressing, and keep it sterile, prior to it being applied to a user's skin 5.

In an further embodiment, the skin treatment dressing is applied to a user's skin and the light from the light emitting part shone through the skin treatment dressing to the user's skin, whilst the light emitting part is not attached to the skin treatment dressing.

In a further embodiment, after a certain period of time of shining light from the light emitting part, through the skin treatment dressing, to a user's skin, the light emitting part is removed from the skin treatment dressing, whilst the skin treatment dressing remains attached to the user's skin for a further period of time.

In a further embodiment, a method of manufacture of a dermatologic treatment apparatus comprise providing a light emitting part for emitting light for dermatological treatment, and attaching a skin treatment dressing to the light emitting part, the skin treatment dressing being configured such that, in use, light from the light emitting part passes through the skin treatment dressing to the skin 5 of a user.

The method of manufacture is repeated, so as to manufacture a plurality of dermatologic treatment apparatus and wherein each skin treatment dressing is cut from the same length of a skin treatment dressing.

It will be appreciated that numerous modifications to the above described design may be made without departing from the scope of the invention as defined in the appended claims.

For example, any of the described embodiments may have any of the features of the other embodiments, in any combination.

For example, in the described embodiments the light transmitting material comprises a plurality of light transmitting regions. Alternatively, the light transmitting material may only have one said light transmitting region. However, it is preferable that the light transmitting material comprises a plurality of said light transmitting regions.

In an alternative version of the seventh embodiment, the light transmitting material 681 may comprise more than two layers, with a continuous first layer upon which two or more layers with apertures are affixed).

The light transmitting material of any of the embodiments shown in Figures 9 and 10 may comprise more than two layers.

Where in the foregoing description, integers or elements are mentioned which have known, obvious or foreseeable equivalents, then such equivalents are herein incorporated as if individually set forth. Reference should be made to the claims for determining the true scope of the present invention, which should be construed so as to encompass any such equivalents. It will also be appreciated by the reader that integers or features of the invention that are described as preferable, advantageous, convenient or the like are optional and do not limit the scope of the independent claims.
Various examples can be defined as set out in any one or more of the following numbered clauses:

### Clauses

1. A dermatologic treatment apparatus comprising:
   a light emitting part, for emitting light for dermatological treatment;
   wherein the apparatus further comprises a skin treatment dressing;
   the skin treatment dressing is a bioactive dressing;
   the skin treatment dressing is arranged to adhere to a user's skin, during use;
   the skin treatment dressing is arranged with the light emitting part such that, in use, light from the light emitting part passes to the skin treatment dressing;
   and wherein the skin treatment dressing is configured such that the light from the light emitting part passes through the skin treatment dressing to a user's skin.
2. A dermatologic treatment apparatus according to clause 1 wherein the skin treatment dressing is a hydrocolloid dressing.
3. A dermatologic treatment apparatus according to either of clause 1 or 2 wherein the skin treatment dressing comprises at least one active agent.
4. A dermatologic treatment apparatus according to any preceding clause, wherein an outer surface of the skin treatment dressing is provided with a removable layer.
5. A dermatologic treatment apparatus according to any preceding clause wherein the skin treatment dressing is releasably attachable to the light emitting part.
6. A dermatologic treatment apparatus according to any preceding clause, comprising a switching apparatus configured to detect the presence of a user's skin and to switch the light emitting part on when the presence of skin is detected.
7. A dermatologic treatment apparatus according to clause 6 wherein the switching apparatus comprises first and second electrodes adapted to switch the light emitting part on when the presence of skin is detected by a change in electrical signal between the two electrodes.
8. A dermatologic treatment apparatus according to any preceding clause, comprising a timer adapted to control the duration that the light emitting part is on, to deliver a predetermined dose of light to the skin of a user.
9. A dermatologic treatment apparatus according to clause 8, when dependent on either of clause 6 or 7, wherein the timer is adapted to activate upon placement of the apparatus onto a user's skin.
10. A dermatologic treatment apparatus according to any preceding clause, comprising a rechargeable battery system configured to power the light emitting part.
11. A dermatologic treatment apparatus according to clause 10 wherein the rechargeable battery system comprises a rechargeable battery and an inductive coupling configured such that the rechargeable battery may be charged by a charger unit by electromagnetic induction.
12. A dermatologic treatment apparatus assembly comprising a dermatologic treatment apparatus according to clause 11 and the charger unit.
13. A dermatologic treatment apparatus according to clause 10, wherein the rechargeable battery system comprises a rechargeable battery configured to be charged by a charging attachment when the charging attachment is attached to the apparatus.
14. A dermatologic treatment apparatus assembly comprising a dermatologic treatment apparatus according to clause 13 and the charging attachment.
15. A dermatologic treatment apparatus according to clause 13 or a dermatologic treatment apparatus assembly according to clause 14 wherein the charging attachment is a charging base.
16. A dermatologic treatment apparatus according to any preceding clause wherein the skin treatment dressing comprises at least one light transmitting region that is configured to provide for greater transmission of light, from the light emitting part to the skin of a user, than another region of the skin treatment dressing.
17. A dermatologic treatment apparatus according to clause 16 wherein the at least one light transmitting region has a substantially circular cross-sectional shape.
18. A dermatologic treatment apparatus according to clause 17 wherein the circular cross-sectional shape has a diameter between 0.01mm and 1mm.
19. A dermatologic treatment apparatus according to clause 18 wherein the circular cross-sectional shape has a diameter between 0.1mm and 0.5mm.
20. A dermatologic treatment apparatus according to any of clause 16 to 19 wherein the at least one light transmitting region is a light transmitting channel.
21. A dermatologic treatment apparatus according to any of clause 16 to 20 wherein the at least one light transmitting region is configured to allow the transmission of light from the light emitting part to the skin of the user without attenuation of the light.
22. A dermatologic treatment apparatus according to clause 21, when dependent on clause 20, wherein the light transmitting channel extends along a longitudinal axis that is perpendicular to the face of the skin treatment dressing.
23. A dermatologic treatment apparatus according to any of clause 16 to 22 wherein the at least one light transmitting region is a region of reduced thickness of the skin treatment dressing.
24. A dermatologic treatment apparatus according to clause 23 wherein the skin treatment dressing is a single layer of skin treatment dressing that has at least one region of reduced thickness to provide said light transmitting region.
25. A dermatologic treatment apparatus according to clause 23 wherein the skin treatment dressing comprises a plurality of layers of skin treatment dressing arranged to provide said at least one region of reduced thickness of the skin treatment dressing.
26. A dermatologic treatment apparatus according to clause 25 wherein the skin treatment dressing comprises a first layer of skin treatment dressing that has a substantially uniform thickness and a second layer of skin treatment dressing that has at least one channel or region of reduced thickness such that the combination of the first and second layers has said at least one light transmitting region.
27. A dermatologic treatment apparatus according to clause 25 wherein the skin treatment dressing comprises first and second layers of skin treatment dressing that each have at least one channel or region of reduced thickness, and wherein the at least one channel or region of reduced thickness in the first and second layers are at least partially offset from each other such that the combination of the first and second layers has said at least one light transmitting region.
28. A dermatologic treatment apparatus according to any of clause 16 to 27 wherein the skin treatment dressing comprises a plurality of said light transmitting regions.
29. A dermatologic treatment apparatus according to clause 28 wherein the light transmitting regions are clustered into a plurality of arrays.
30. A dermatologic treatment apparatus according to clause 29 wherein the plurality of arrays are evenly distributed across the surface of the skin treatment dressing.
31. A dermatologic treatment apparatus according to clause 29 wherein the light emitting part comprises a plurality of light sources and wherein the arrays are each clustered around a respective light source.
32. A dermatologic treatment apparatus according to any of clause 28 to 31 wherein the light transmitting regions are distributed in a hexagonal configuration.
33. A dermatologic treatment apparatus according to clause 32 wherein the light transmitting regions are distributed in a hexagonal configuration at intervals between 0.05mm and 2mm apart.
34. A dermatologic treatment apparatus according to clause 33 wherein said intervals are between 0.5mm and 1mm apart.
35. A method of dermatological treatment comprising providing a dermatologic treatment apparatus comprising:
   a light emitting part, for emitting light for dermatological treatment;
   wherein the apparatus further comprises a skin treatment dressing,
   the skin treatment dressing is a bioactive dressing;
   the skin treatment dressing is arranged to adhere to a user's skin, during use;
   and wherein the skin treatment dressing is configured such that the light from the light emitting part passes through the skin treatment dressing to a user's skin;
   wherein the method comprises:
      applying the skin treatment dressing to a skin surface to be treated and transmitting light from the light emitting part through the skin treatment dressing to the skin surface.
36. A method according to clause 35 wherein the skin treatment dressing is arranged with the light emitting part such that, in use, light from the light emitting part passes to the skin treatment dressing.
37. A method according to either of clause 35 or 36 wherein the skin treatment dressing is applied to the skin surface, and the light is transmitted from the light emitting part, through the skin treatment dressing to the skin surface, for a period of at least one minute.
38. A method according to clause 37 wherein the skin treatment dressing is applied to the skin surface, and the light is transmitted from the light emitting part, through the skin treatment dressing to the skin surface, for a period of at least one hour.
39. A method according to any of claims 35 to 38, wherein an outer surface of the skin treatment dressing is provided with a removable layer and wherein the removeable layer is removed prior to applying the skin treatment dressing to the skin surface.
40. A method of manufacture of a dermatologic treatment apparatus, comprising:
   providing a light emitting part, for emitting light for dermatological treatment;
   providing a skin treatment dressing,
   wherein the skin treatment dressing is a bioactive dressing;
   and the skin treatment dressing is arranged to adhere to a user's skin, during use;
   arranging the skin treatment dressing with the light emitting part such that, in use, light from the light emitting part passes to the skin treatment dressing;
   and configuring the skin treatment dressing such that the light from the light emitting part passes through the skin treatment dressing to a user's skin.
41. A method according to clause 40 wherein the method of manufacture is repeated, so as to manufacture a plurality of dermatologic treatment apparatus and wherein each skin treatment dressing is cut from the same length of a skin treatment dressing.
42. A kit of parts comprising:
   a light emitting part, for emitting light for dermatological treatment; and
   a skin treatment dressing,
   wherein the skin treatment dressing is a bioactive dressing;
   the skin treatment dressing being arranged to adhere to a user's skin, during use;
   the skin treatment dressing is for being arranged with the light emitting part such that, in use, light from the light emitting part passes to the skin treatment dressing;
   and wherein the skin treatment dressing is for being configured such that the light from the light emitting part passes through the skin treatment dressing to a user's skin.

## Claims

1. A dermatologic treatment apparatus comprising:
a light emitting part, for emitting light for dermatological treatment;
wherein the apparatus further comprises a skin treatment dressing;
the skin treatment dressing is a bioactive dressing;
the skin treatment dressing is arranged to adhere to a user's skin, during use;
the skin treatment dressing is arranged with the light emitting part such that, in use, light from the light emitting part passes to the skin treatment dressing;
and wherein the skin treatment dressing is configured such that the light from the light emitting part passes through the skin treatment dressing to a user's skin;
wherein the skin treatment dressing comprises at least one light transmitting region that is configured to provide for greater transmission of light, from the light emitting part to the skin of a user, than another region of the skin treatment dressing;
wherein the at least one light transmitting region has a substantially circular cross-sectional shape, wherein the circular cross-sectional shape has a diameter between 0.01mm and 1mm;
wherein the apparatus is flexible, and
the apparatus is less than 2mm thick.

2. A dermatologic treatment apparatus according to claim 1 wherein the apparatus has a general tear drop shape.

3. A dermatologic treatment apparatus according to any preceding claim wherein the skin treatment dressing is a hydrocolloid dressing.

4. A dermatologic treatment apparatus according to any preceding claim, wherein an outer surface of the skin treatment dressing is provided with a removable layer.

5. A dermatologic treatment apparatus according to any preceding claim wherein the skin treatment dressing is releasably attachable to the light emitting part.

6. A dermatologic treatment apparatus according to any preceding claim, comprising a rechargeable battery system configured to power the light emitting part.

7. A dermatologic treatment apparatus according to any preceding claim, wherein the at least one light transmitting region is a light transmitting channel.

8. A dermatologic treatment apparatus according to any preceding claim wherein the at least one light transmitting region is a region of reduced thickness of the skin treatment dressing.

9. A dermatologic treatment apparatus according to any preceding claim wherein the skin treatment dressing comprises a plurality of said light transmitting regions, wherein the light transmitting regions are clustered into a plurality of arrays, and wherein the light emitting part comprises a plurality of light sources and the arrays are each clustered around a respective light source.

10. A dermatologic treatment apparatus according to any preceding claim wherein the skin treatment dressing comprises a plurality of said light transmitting regions distributed in a hexagonal configuration at intervals between 0.05mm and 2mm apart.

11. A method of manufacture of a dermatologic treatment apparatus, comprising:
providing a light emitting part, for emitting light for dermatological treatment;
providing a skin treatment dressing,
wherein the skin treatment dressing is a bioactive dressing;
and the skin treatment dressing is arranged to adhere to a user's skin, during use;
arranging the skin treatment dressing with the light emitting part such that, in use, light from the light emitting part passes to the skin treatment dressing;
and configuring the skin treatment dressing such that the light from the light emitting part passes through the skin treatment dressing to a user's skin;
wherein the skin treatment dressing comprises at least one light transmitting region that is configured to provide for greater transmission of light, from the light emitting part to the skin of a user, than another region of the skin treatment dressing;
wherein the at least one light transmitting region has a substantially circular cross-sectional shape, wherein the circular cross-sectional shape has a diameter between 0.01mm and 1mm;
wherein the apparatus is flexible, and
the apparatus is less than 2mm thick.

12. A method according to claim 11 wherein the apparatus has a general tear drop shape.

13. A method according to either of claims 11 or 12 wherein the method of manufacture is repeated, so as to manufacture a plurality of dermatologic treatment apparatus and wherein each skin treatment dressing is cut from the same length of a skin treatment dressing.

14. A kit of parts comprising:
a light emitting part, for emitting light for dermatological treatment; and
a skin treatment dressing,
wherein the skin treatment dressing is a bioactive dressing;
the skin treatment dressing being arranged to adhere to a user's skin, during use;
the skin treatment dressing is for being arranged with the light emitting part such that, in use, light from the light emitting part passes to the skin treatment dressing;
and wherein the skin treatment dressing is for being configured such that the light from the light emitting part passes through the skin treatment dressing to a user's skin;
wherein the skin treatment dressing comprises at least one light transmitting region that is configured to provide for greater transmission of light, from the light emitting part to the skin of a user, than another region of the skin treatment dressing;
wherein the at least one light transmitting region has a substantially circular cross-sectional shape, wherein the circular cross-sectional shape has a diameter between 0.01mm and 1mm;
wherein the apparatus is flexible, and
the apparatus is less than 2mm thick.

15. A kit of parts according to claim 14 wherein the apparatus has a general tear drop shape.
